# DEMANDE DE BREVET EUROPEEN

(11) **EP 2 690 169 A1**
(43) Date de publication de la demande: **29.01.2014**
(21) Numéro de dépôt: 13305835.4
(22) Date de dépôt: 20.06.2013
(51) Int. Cl.: C12N 1/20, A62D 3/02, C12R 1/01

(54) **Bactéries du genre Pseudoxanthomonas capables de dégrader le méthyl tert-butyl éther (MTBE) en solution dans des effluents**

(30) Priorité: 25.07.2012 FR 1202104
(71) Demandeur: IFP Energies nouvelles, 92852 Rueil-Malmaison Cedex (FR)
(72) Inventeur: Le Digabel, Yoann, 92800 Puteaux (FR); Fayolle-Guichard, Francoise, 75005 Paris (FR)

(57) **Abrégé**

La présente invention concerne des bactéries du genre *Pseudoxanthomonas* et en particulier la souche déposée le 12 juillet 2012 à l'Institut Pasteur (CNCM, 25 rue du Docteur Roux, F-75724 PARIS Cedex 15, France) sous la n° CNCM I-4657, capables de dégrader le MTBE et/ou le TBA.

L'invention porte également sur un procédé de traitement d'un effluent comprenant du MTBE et éventuellement du TBA mettant en oeuvre de telles bactéries.

## Description

La présente invention concerne des microorganismes du genre capables de dégrader des additifs des essences et en particulier le méthyl tert-butyl éther (MTBE) ou le tert-butyl alcool (TBA) en solution dans l'eau. Les microorganismes selon l'invention trouvent leur application dans l'industrie du traitement des eaux._La présente invention a également pour objet un procédé de traitement d'effluents aqueux contenant du méthyl tert-butyl éther (MTBE) et/ou du tert-butyl alcool (TBA) mettant en oeuvre de telles bactéries.

### Etat de la technique

Il est connu que des additifs sont ajoutés aux essences pour améliorer les performances des moteurs, c'est le cas des additifs oxygénés, ou éthers-carburants : le méthyl *tert*-butyl éther (désigné ci-après sous le terme de MTBE) est un des éthers qui peut être utilisé comme additif oxygéné dans les essences sans plomb dans le but d'augmenter leur indice d'octane ainsi que de l'éthyl *tert*-butyl éther (désigné ci-après sous le terme d'ETBE) qui est préférentiellement utilisé depuis plusieurs années en France et en Europe du fait de sa qualification en tant que biocarburant. Ces composés peuvent être ajoutés aux essences à raison de 22% (v/v).

Le transport d'hydrocarbures, par voie terrestre ou maritime, présente de nombreux risques d'accidents. Le transport par pipe-lines qui est généralement considéré comme plus sûr que par camion, train ou tanker peut néanmoins induire des pollutions. Il a été estimé en 2010 que les pipelines représentent la source la plus fréquente (27 %) des déversements. Les pollutions terrestres par les hydrocarbures sont également dues à des accidents de camions ou de trains pendant le transport, des accidents durant le remplissage des cuves de stations-service, des fuites sur des cuves de stockage dans des stations-service ou sur des sites industriels. En plus de ces sources majeures de pollution par hydrocarbures, il existe une pollution chronique qui se produit durant le remplissage des réservoirs des véhicules dans les stations-service ou à des fuites sur les réservoirs des véhicules. Dans ces deux derniers cas, cette décharge vers les eaux terrestres de très faible quantité de manière chronique est aussi importante.

Parmi les composés des essences, tous n'ont pas la même toxicité et/ou biodégradabilité et ceci va déterminer leur devenir dans l'environnement. Le benzène, par exemple, qui est un des composés monoaromatiques des essences, est un composé très toxique mais facilement dégradé en aérobiose. Parmi les composés natifs des essences qui sont récalcitrants à la biodégradation, on peut citer le 2,2,4-triméthylpentane (désigné sous le terme d'isooctane) ou le cyclohexane, dont les niveaux de toxicité sont un peu moindres.

La littérature relative à la biodégradation des composés des essences ou des alcanes par les microorganismes est importante et de nombreux microorganismes avec des capacités de dégradation de ces composés ont été isolés. Par contre, un nombre plus restreint de microorganismes avec des capacités de dégradation du MTBE ou du TBA, dont la biodégradation est plus lente que celle des composés dit "facilement biodégradables", ont été isolés.

En raison de l'utilisation croissante d'additifs tels que le MTBE dans les formulations d'essences ou de gazole, il est donc nécessaire de connaître le devenir de ces composés en cas de déversement accidentel conduisant à une pollution des sols et des eaux souterraines ou de surface. Cette nécessité est d'autant plus grande dans le cas du MTBE car ce composé est très soluble dans l'eau (40 g.L⁻¹) et qu'il est considéré comme potentiellement toxique et que, hormis toute considération de toxicité, sa présence dans l'eau à de très faibles concentrations rend l'eau impropre à la consommation du fait du goût qu'il lui confère.

Un but de l'invention est de proposer de nouveaux microorganismes capables de biodégrader le MTBE et éventuellement du TBA qui peuvent atteindre les nappes aquifères dans les cas de pollution.

### Résumé de l'invention

La Demanderesse a de façon surprenante trouvé que les bactéries du genre *Pseudoxanthomonas* et en particulier la souche déposée le 12 juillet 2012, à l'Institut Pasteur (CNCM, 25 rue du Docteur Roux, F-75724 PARIS Cedex 15, France) sous le n° CNCM I-4657 avaient des capacités de biodégradation importantes du MTBE et du TBA.

### Description détaillée de l'invention

La Demanderesse a observé que lorsque l'on fournit à la bactérie du genre *Pseudoxanthomonas,* et en particulier la souche déposée, le 12 juillet 2012 à l'Institut Pasteur (CNCM, 25 rue du Docteur Roux, F-75724 PARIS Cedex 15, France) sous le n° CNCM I-4657, du MTBE, celle-ci s'est avérée capable de le dégrader en produisant du *tert-*butyl alcool (TBA) comme composé intermédiaire de la biodégradation qui est ensuite totalement consommé par cette bactérie. Cette observation montre que la bactérie du genre *Pseudoxanthomonas* possède la totalité des enzymes de dégradation permettant d'aller jusqu'à la minéralisation et la production de biomasse.

Les bactéries selon l'invention ont été isolées à partir de microcosmes provenant de différents environnements qui ont été obtenus par enrichissement sur un milieu minimum contenant le MTBE comme seule source de carbone. Ce protocole a été réalisé selon les techniques d'enrichissement en microorganismes spécifiques connues de l'homme de l'art et est une méthode permettant de sélectionner les bactéries capables de dégrader le MTBE.

Les souches bactériennes résultantes sont isolées après ces étapes d'enrichissement spécifiques sur des boîtes de pétri contenant du milieu riche classiquement utilisé par l'homme de l'art (milieu Tripticase/Soja ou TS) mais après dilution de ce milieu au 1/10 par rapport à la concentration habituellement utilisée. Ces bactéries ont ensuite été identifiées d'après leur séquence d'ADNr 16S et par comparaison avec les banques de données des ADN bactériens puis elles ont été testées pour leurs capacités de dégradation du MTBE.

La présente invention concerne également un procédé de traitement d'effluents contenant du MTBE et éventuellement du TBA dans lequel on met en contact en conditions aérobies l'effluent en présence au moins d'une bactérie du genre *Pseudoxanthomonas* déposée le 12 juillet 2012 à l'Institut Pasteur (CNCM, 25 rue du Docteur Roux, F-75724 PARIS Cedex 15, France) sous le n° CNCM I-4657. Lamise en oeuvre de ces bactéries pour le traitement en continu d'effluents pollués par du MTBE peut être réalisée par exemple dans un biofiltre où les bactéries sont fixées sur un support minéral ou organique ou bien elles peuvent être ajoutées comme inoculum à des boues de station d'épuration, ou dans tout autre système adapté au traitement des eaux et des sols (biobarrière).

Selon un mode de réalisation avantageux, le procédé de traitement peut mettre en oeuvre les bactéries selon l'invention en association avec les bactéries décrites dans la demande de brevet FR 2 944 006, lorsque l'effluent contient un cocktail de composés hydrocarbonés tels que l'octane, le benzène, l'éthylbenzène, le toluène, le *m*-xylène, le p-xylène, le cyclohexanol, le cyclohexane, l'isooctane.

### Brève description des figures

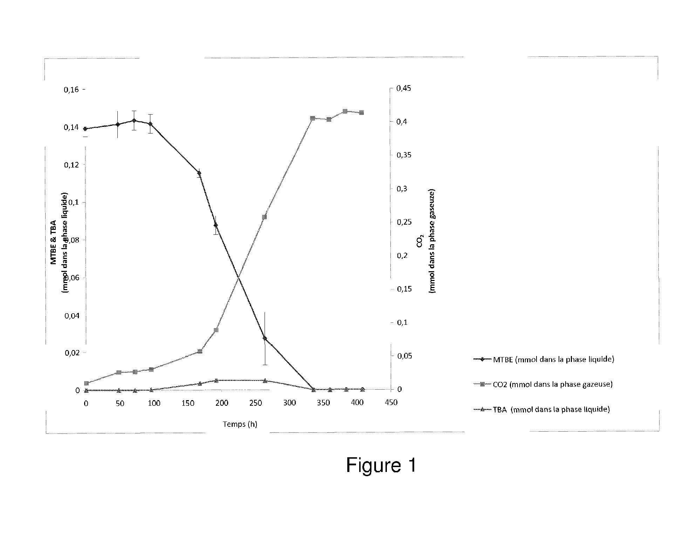

Ces aspects ainsi que d'autres aspects de l'invention seront clarifiés dans la description détaillée de modes de réalisation particuliers de l'invention, référence étant au dessin de la figure 1, dans laquelle la figure 1 montre l'évolution de la concentration en MTBE, en TBA dans le milieu culture et du CO₂ formé en fonction du temps de contact avec la bactérie du genre *Pseudoxanthomonas* CNCM I-4657.

### Exemples

### Croissance de la bactérie du genre Pseudoxanthomonas CNCM I-4657 sur un milieu minéral en présence de MTBE comme seule source de carbone

On effectue une préculture de la bactérie du genre *Pseudoxanthomonas* CNCM I-4657 : on ensemence la souche *Pseudoxanthomonas* CNCM I-4657 sur milieu minéral salin MM supplémenté en MTBE à environ 200 mg.L⁻¹ comme source de carbone et d'énergie.

Le milieu MM a la composition suivante :

| | |
|---|---|
| KH₂PO₄ | 1,4 g |
| K₂HPO₄ | 1,7 g |
| NaNO₃ | 1,5 g |
| MgSO₄, 7H₂O | 0,5 g |
| CaCl₂, 2H₂O | 0,04 g |
| FeCl₃, 6H₂O | 0,012 g |
| Solution concentrée de vitamines | 1 mL |
| Solution concentrée d'oligoéléments | 1 mL |
| H₂O | 1 L |

La solution concentrée de vitamines a la composition suivante pour 1 litre d'eau distillée :

| | |
|---|---|
| Biotine | 200 mg |
| Riboflavine | 50 mg |
| Acide nicotinamique | 50 mg |
| Panthoténate | 50 mg |
| Acide p-aminobenzoïque | 50 mg |
| Acide folique | 20 mg |
| Thiamine | 15 mg |
| Cyanocobalamine | 1,5 mg |

La solution concentrée d'oligoéléments a la composition suivante pour 1 litre d'eau distillée :

| | |
|---|---|
| CuSO₄, 5 H₂O | 0,1 g |
| MnSO₄, 2H₂O | 1 g |
| ZnSO₄, 7 H₂O | 1 g |
| AlCl₃, 6H₂O | 0,4 g |
| NiCl₂,6 H₂O | 0,25 g |
| H₃BO₃ | 0,1 g |
| CoCl₂, 6 H₂O | 1 g |
| Na₂MoO₄, 2H₂O | 1 g |
| Na₂WO₄, 2H₂O | 1 g |

Après croissance des boites, cette culture sur milieu solide est utilisée pour ensemencer 150 mL de milieu minéral salin tel que décrit dans l'exemple (les colonies sont raclées avec une öse et directement introduites dans le milieu de culture comme inoculum) auquel on ajoute du MTBE à une concentration finale d'environ 300 mg.L⁻¹ dans une fiole d'Erlenmeyer d'une contenance de 500 mL fermée avec un bouchon téflonné afin d'éviter toute perte de MTBE au cours de la croissance. Un prélèvement est effectué au temps t=0 pour un dosage du MTBE au départ par analyse en chromatographie en phase gazeuse avec un détecteur à ionisation de flamme (CPG/FID). La fiole est ensuite incubée à 30°C dans un agitateur rotatif. Un prélèvement pour le dosage du substrat et de ses éventuels produits de dégradation est effectué à intervalle régulier. La production de CO₂ produit dans la phase gazeuse est également mesurée par prélèvement de ladite phase gazeuse à travers le septum avec une seringue à gaz étanche par analyse du CO₂ en chromatographie en phase gazeuse avec un détecteur de type catharomètre (CPG/TCD).

Le résultat de cette expérimentation est présenté dans la figure 1. Comme on le voit sur cette figure 1 le MTBE est dégradé en partie en TBA à faible concentration. Ce composé est ensuite lui-même re-consommé et utilisé comme substrat de croissance.

On peut appliquer le protocole décrit ci-dessus à différentes bactéries pour évaluer leur capacité a dégrader le MTBE et donc permet de sélectionner les bactéries capables de dégrader le MTBE.

## Revendications

1. Bactérie du genre *Pseudoxanthomonas* capable de dégrader du MTBE et/ou du TBA.

2. Bactérie du genre *Pseudoxanthomonas* déposée le 12 juillet 2012 à l'Institut Pasteur (CNCM, 25 rue du Docteur Roux, F-75724 PARIS Cedex 15, France) sous le n° CNCM I-4657.

3. Procédé de traitement d'un effluent comprenant du MTBE et éventuellement du TBA comme substrat de croissance dans lequel on met en contact, dans des conditions aérobies, ledit effluent avec au moins une bactérie selon la revendication 2.

4. Procédé selon la revendication 3 dans lequel la bactérie est fixée dans un biofiltre

5. Procédé selon la revendication 4 dans lequel la bactérie est fixée sur un support minéral ou organique.

6. Procédé selon la revendication 3 dans lequel on met en contact l'effluent avec une boue d'épuration dans laquelle a été inoculée une bactérie selon la revendication 2.
